**Europäisches Patentamt**

**European Patent Office**

**Office européen des brevets**

(19)

(11) Publication number : **0 514 056 A2**

(12)

# EUROPEAN PATENT APPLICATION

(21) Application number : **92303998.6**

(22) Date of filing : **01.05.92**

(51) Int. Cl.$^5$ : **A61K 31/00, A61K 31/56, A61K 31/565**

(30) Priority : **03.05.91 US 695164**
**03.05.91 US 695196**
**03.05.91 US 695197**

(43) Date of publication of application :
**19.11.92 Bulletin 92/47**

(84) Designated Contracting States :
**CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Elbrecht, Alex**
**65 Maple Street**
**Watchung, NJ 07060 (US)**
Inventor : **Smith, Roy G.**
**528 Forest Avenue**
**Westfield, NJ 07090 (US)**

(74) Representative : **Thompson, John Dr. et al**
**Merck & Co., Inc. European Patent**
**Department Terlings Park Eastwick Road**
**Harlow, Essex CM20 2QR (GB)**

(54) Use of estrogens or antiandrogens for reversal of male sexual phenotype in poultry.

(57)     Fertilized poultry embryos are treated with estrogens or antiandrogens or estrogens in combination with antiandrogens during a period of embryonic development when the gonads are bipotential. This treatment causes genetic males to exhibit a female phenotype. When sex reversed male birds are crossed with normal male birds all offspring will be genotypically male.

EP 0 514 056 A2

## BACKGROUND OF THE INVENTION

Males of commercially important poultry species gain weight faster during the first eight weeks after hatching than do females of the same species, Nixey, 7th Eur. Poultry Conf., Vol. 2, Larbier, ed., pg. 671-679 (1988). This weight gain advantage allows male birds to reach a marketable weight before females. A uniform weight gain of both male and female birds would have a significantly positive economic impact on broiler production for all important species.

Both female-to-male and male-to-female sex reversal has been achieved in several species of fish, Hunter and Donaldson, Fish Physiology, IXB: 223-303 (1983). This was usually accomplished by treatment with gonadal steroids during a labile period of embryonic development.

Female-to-male sex reversal has been accomplished in poultry by grafting of female embryos with embryonic testes, Stoll et al., Gen. Comp. Endocrinol. 41:66-75 (1980). A permanent male-to-female sex reversal has not been described for poultry although the feminizing effects of treatment with estrogens have been documented. Teng and Teng, *Ontogeny of Receptors and Reproductive Hormone Action*, eds. T.H. Hamilton, J.H. Clark and W.A. Sadler, Raven Press, New York, 1979, have shown that early treatment of embryos with the synthetic estrogen diethylstilbestrol prevents regression of the Mullerian ducts in males. Sayag *et al.*, Physiology & Behavior 45: 1107-1112 (1989), treated chicken embryos with estradiol on day 10 of incubation and demonstrated a feminization of masculine sexual behavior at puberty. Treatment on day 10 of incubation is too late to affect sex determination but apparently not too late to affect sexual behavior.

## OBJECTS OF THE INVENTION

It is, accordingly, an objective of the present invention to provide a means of converting genotypic male birds into phenotypic female birds. Another object is to treat bird embryos with estrogen to convert male genotypic birds into female phenotypic birds. Another object is to treat bird embryos with antiandrogen to convert male genotypic birds into female phenotypic birds. Another object is to treat bird embryos with an estrogen in combination with antiandrogen to convert male genotypic birds into female phenotypic birds. Another object is to prepare strains of fowl which produce only male fowl. A further object is to obtain phenotypically female birds with a male genotype. Another object is to be able to irreversibly change the sexual phenotype of birds by treating a bird embryo with estrogens in combination with antiandrogens prior to about day 9 of embryonic development.

## SUMMARY OF THE INVENTION

Fertilized poultry embryos are treated with estrogens or antiandrogens or estrogens in combination with antiandrogens during a period of embryonic development when the gonads are bipotential. This treatment causes genetic males to exhibit a female phenotype. When sex reversed male birds are crossed with normal male birds all offspring will be genotypically male.

## DETAILED DESCRIPTION OF THE INVENTION

The present invention relates to the conversion of a genotypic male fowl to a phenotypic female fowl. Fowl is defined herein as wild or domesticated gallinaceous birds that serve as a source of meat and that include, but are not limited to, commercially important birds such as chickens, turkeys, ducks, geese, guinea fowl, pheasants, pigeons, peafowl and quail. Fowl as used herein will also include Poultry, all domesticated birds kept for meat.

Fertilized embryos of any domesticated gallinaceous bird, with chicken, goose, duck and turkey being preferred, are treated with one or more estrogens, one or more androgens or estorgens in combination with one or more antiandrogens at a point in development which will allow the conversion of a male genotype to a female phenotype. Estrogen as used herein is defined as any substance, natural or synthetic, that exerts biological effects characteristic of estrogenic hormones such as estradiol. Estrogens are well known in the art, as are methods for obtaining them. Representative steroidal estrogens include, but are not limited to, the following which are commercially available from a number of sources: 17 β-estradiol, (17 β)-Estra-1,3,5(10)-triene-3,17-diol; estradiol benzoate, (17 β)-Estra-1,3,5(10)-triene-3,17-diol-3-benzoate; estradiol 17 β-cypionate, (17 β)-estra-1,3,5(10)-triene-3,17-diol-17-cyclopentanepropanoate; estrone, 3-hydroxyestra-1,3,5(10)-trien-17-one, 1,3,5-estratriene-3,17 α-diol and estriol, estra-1,3,5(10)-triene-3,16,17-triol. Representative non-steroidal estrogens include, but are not limited to, chlorotris (p-methoxyphenyl) ethylene, 4,4'-(diethylideneethylene) diphenol, trans-4,4'-dihydroxy-α β-diethylstilbene, 17 α-ethynylestra-1,3,5(10)-triene-3,17β-diol and 3-methoxy-19,nor-17 α-pregna-1,3,5(10)-triene-20-yn-17-ol which are know in the art and commercially available.

Antiandrogen as used herein is defined as any substance, natural or synthetic, that inhibits the synthesis or action of androgens. Representative antiandrogens are listed below along with a cited reference. The references will directly contain a method for making the compound or will direct one who wishes to use the compound to a method for producing the compound. Typical suitable antiandrogens known in the art and useful and included within the scope of this invention include nonsteroidal antiandrogens such as the imidazolidines, especially 1-(3'-trifluoromethyl- 4'nitrophenyl)-4,4-dimethyl-imidazoline-2,5-dione (also called Anandron) described in U.S. Pat. 4,097,578; 4'-nitro-3-trifluoromethylisobutyranilide (also called flutamide) described in U.S. Pat. 3,847,988, hydroxyflutamide described in U.S. Pat. 3,875,229 and prodrug forms of hydroxyflutamide; the N-(phenylalkanoyl)aniline derivatives disclosed in U.S. Pat. 4,386,080; the 3,4-disubstituted--branched--chain acylanilides disclosed in U.S. Pat. 4,239,776 (A.T. Glen, et al.) and specifically casodex (Imperial Chemical Industries), which is N'-[4-cyano-3-(trifluoromethyl) phenyl]-3-t(4-fluorophenyl)sulfonyl]-2-hydroxy-2-methyl-propionamide disclosed in U.S. Pat. 4,636,505 (H. Tucker).

Representative compounds of the non-steroidal antiandrogens described above are the following:

1-(3'-trifluoromethyl1-4'-nitrophenyl)-4,4-dimethyl-5-iminoimidazoline-2-one;
1-(3'-trifluoromethyl1-4'-nitrophenyl)-4,4-dimethyl-1imidazoline-2,5-dione
1-(3'-trifluoromethyl-4'-nitrophenyl)-4,4-dimethyl-imidazoline-2,5-dione;
ethyl N-(4-nitro-3-trifluoromethylphenyl)-cyclopropylcarbimidate;
ethyl N-(4-nitro-3-trifluoromethylphenyl)-isobutyrimidate;
ethyl N-(4-iodo-3-trifluoromethylphenyl)-isobutyrimidate;
ethyl N-(4-nitro-3-trifluoromethylphenyl)-2.3-dimethyl-butyrimidate;
ethyl N-(4'-nitro-3'-trifluoromethylphenyl)-2-methylbutyrimidate;
ethyl N-(4-bromo-3-trifluoromethylphenyl)-isobutyrimidate;
ethyl N-(4-nitrophenyl)-isobutyrimidate;
ethyl N-(4-chloro-3-trifluoromethylphenyl)-isobutyrimidate;
ethyl N-(3-bromo-4-nitrophenyl)-isobutyrimidate;
ethyl N-(3-chloro-4-nitrophenyl)-isobutyrimidate;
N-(4-Nitro-3-trifluoromethylphenyl)-isobutyrchlorimidate;
4-Nitro-3-trifluoromethylisobutyrthioanilide;
Ethyl N-(4-nitro-3-trifluoromethylphenyl)-isobutyrimidate;
Methyl N-(4-nitro-3-trifluoromethylphenyl)-2-hydroxyisobutyrimidate;
2-bromo-4'-nitro-3'-trifluoromethylisobutyranalide;
2-methoxy,2-fluoro-2-chloro-3'-trifluoromethylisobutyranilide;
2-iodo-4'-nitro-3'trifluoromethylisobutyranilide;
N-methyl-2-bromo-4'-nitro-3'-trifluoromethylisobutyranilide;
N-ethyl-2-bromo-4'-nitro-3'-trifluoromethylisobutyranilide;
N-propyl-2-bromo-4'-nitro-3'-trifluoromethylisobutyranilide;
N-butyl-2-bromo-4'-nitro-3'-trifluoromethylisobutyranilide;
2-hydroxy-4'-nitro-3'-trifluoromethylisobutyranilide;
3',4'-dinitro-2-hydroxyisovaleranilide;
4'-chloro-2-hydroxy-3'-trifluoromethylcyclobutylcarbanilide;
3'-chloro-2-hydro-4'-iodocyclopropylcarbanilide;
3'-bromo-2-hydroxy-3-methyl-4'-nitrovaleranilide;;
2-hydroxy-3'-iodo-4'-trifluoromethylisobutyranilide;
2,3-dimethyl-2-hydroxy-3'-methyl-4'-nitro-aleranilide;
3'-acetyl-2,3-dimethyl-2-hydroxy-4'-iodovaleranilide;
3'-acetyl-2,3-dimethyl-2-hydroxy-4'-nitrovaleranilide;
2-hydroxy-3'-methoxy-4'nitroisobutyranilide;
3'-ethyl-2-hydroxy-4'-trifluoromethylisobutyranilide;
2,3-dimethyl-2-hydroxy-4'-nitro-3'-trifluoromethylthiovaleranilide;
4'-chloro-2-hydroxy-3'-propylisobutyranilide;
3'-bromo-2,3-dimethyl-2-hydroxy-4'-trifluoromethylcyclopropylcarbanilide;
2-hydroxy-4'-nitrobutyranilide;
2-hydroxy-3'-nitro-4'-bromoisobutyranilide;
4'-chloro-2-hydroxy-3'-iodoisovaleranilide;
2-bromo-4'-nitrocyclopropylcarbanilide;
2,3-dimethyl-2-hydroxy-3'-propionyl-4'-trifluoromethylbutyranilide;
2,3-dimethyl-2-hydroxy-3'-propionyl-4'-nitrobutyranilide;

2-hydroxy-4'-trifluoromethylisobutyranilide;
 2-hydroxy-2-methyl-4'-nitro-3'-trifluoromethylbutyranilide;
2-hydroxy-3--4'-dichloroisobutyranilide;
2-hydroxy-3'-4'-diiodoisobutyranilide;
3'-fluoro-2-hydroxy-4'-nitroisobutyranilide;
2-hydroxy-4'-chloro-3'-trifluoromethylisobutyranilide;
2-hydroxy-4'-nitroisobutyranilide;
2-ethyl-2-hydroxy-4'-nitro-3'-trifluoromethylbutyranilide;
3'-bromo-2-hydroxy-4'-nitroisobutyranilide;
2,3-dimethyl-2-hydroxy-4'-nitro-3'-trifluoromethylbutyranilide;
2-hydroxy-N-methyl-4'-nitro-3'-trifluoromethylisobutyranilide;
4'-chloro-2-hydroxy-N-methyl-3'-trifluoromethylisobutyranilide;
4'-bromo-2-hydroxy-3'-trifluoromethylisobutyranilide;
2,3-dimethyl-2-hydroxy-4'-nitro-3'-trifluoromethylbutyranilide;
2-hydroxy-N-methyl-4'-nitro-3'-trifluoromethylisobutyranilide;
2-acetoxy-4'-nitro-3'-trifluoromethylisobutyranilide;
2-methoxy-4'-nitro-3'-trifluoromethylisobutyranilide;
2-valeryloxy-4'-nitro-3'-trifluoromethylisobutyranilide;
2-hydroxy-3'-bromo-4'-nitroisobutyranilide;
2-hydroxy-4'-bromo-3'-trifluoromethylisobutyranilide;
2-acetoxy-4'-bromo-3'-trifluoromethylisobutyranilide;
3'-bromo-2-chloro-4'-nitroisobutyranilide;
2-bromo-4'-nitro-3'-trifluoromethylisobutyranilide;
N-methyl-2-methoxy-4'-nitro-3'-trifluoromethylisobutyranilide;
4'-bromo-2-chloro-3'-trifluoromethylisobutyranilide;
2-chloro-4'-iodo-3'-trifluoromethylisobutyranilide;
2-acetoxy-4'-bromo-3'-trifluoromethylisobutyranilide;
3'-chloro-2-hydroxy-4'-nitroisobutyranilide;
4'-bromo-2-methoxy-3'-trifluoromethylisobutyranilide;
2-chloro-4'-nitro-3'-trifluoromethylisobutyranilide;
2-hydroxy-4'-chloro-3'-trifluoromethylisobutyranilide;
2-hydroxy-4'-nitro-3'-trifluoromethylcyclopropylcarbanilide;
2-hydroxy-4'-iodo-3'-trifluoromethylcyclopropylcarbanilide;
2,3-dimethyl-2-hydroxy-4'-nitro-3'-trifluoromethylbutyranilide;
2-hydroxy-4'-nitro-3'-trifluoromethylisovaleranilide;
4'-bromo-2-hydroxy-3'-trifluoromethylisobutyranilide;
2'-hydroxy-4'-nitroisobutyranilide;
4'-chloro-2-hydroxy-3'-trifluoromethylisobutyranilide;
3'-bromo-2-hydroxy-4'-nitroisobutyranilide;
3'-chloro-2-hydroxy-4'-nitroisobutyranilide;
2-chloro-4'-nitro-3'-trifluoromethylcyclopropylcarbanilide;
2-chloro-4'nitro-3'-iodo-3'-trifluoromethylbutyranilide;
2-chloro-4'-nitro-3'-trifluoromethylcyclopropylcarbanilide;
2-chloro-4'-nitro-3'-trifluoromethyl-isobutyranilide;
2-chloro-2,3-dimethyl-4'iodo-3'-trifluoromethylbutyranilide;
2-chloro-4'-nitro-3'-trifluoromethyli~ovaleranilide;
4'-bromo-2-chloro-3'-trifluoromethyli~ovaleranilide;
4'-bromo-2-chloro-3'-trifluoromethylisobutyranilide;
2,4'-dichloro-3'-trifluoromethylisobutyranilide;
3'-bromo-2-chloro-4'-nitroisobutyranilide;
2,3'-dichloro-4'-nitroisobutyranilide;
4'-nitro-3'-trifluoromethyl-2-valeryloxyisobutyranilide;
3,4-dichloro-N-(2-hydroxy-2-p-nitrophenylpropionyl)aniline;
4-nitro-3-trifluoromethyl-N-(2-hydroxy-2-p-nitro-phenylpropionyl)aniline;
4-cyano-3-trifluoromethyl-N-(2-hydroxy-2-p-nitro-phenylpropionyl)aniline;
3-chloro-4-nitro-N-(2-hydroxy-2-p-nitrophenylpropionyl)aniline;
3-chloro-4-methylsulphonyl-N-(2-hydroxy-2-p-nitro-phenylpropionyl)aniline;
4-nitro-3-trifluoromethyl-N-(2-hydroxy-2-p-trifluoromethylphenylpropionyl)aniline;

3-chloro-4-nitro-N-(2-hydroxy-2-p-trifluoromethylphenylpropionyl)aniline;
4-nitro-3-trifluoromethyl-N-(2-p-cyanophenyl-2-hydroxypropionyl)aniline;
 4-nitro-3-trifluoromethyl-N-(2-p-fluorophenyl-2-hydroxypropionyl)aniline;
4-nitro-3-trifluoromethyl-N-(2-hydroxy-2-p-methylsulphinylphenylpropionyl)aniline;
3,4-dichloro-N-(2-hydroxy-2-p-methylsulphinylphenylpropionyl)aniline;
3,4-dichloro-N-(2-p-acetylphenyl-2-hydroxypropionyl)aniline;
3,4,5,-trichloro-N-(2-hydroxy-2-p-nitrophenylpropionyl)aniline;
2-chloro-4-nitro-N-(2-hydroxy-2-p-nitrophenylpropionyl)aniline;
N-(2-methoxy-2-p-nitrophenylpropionyl)-4-nitro-3-trifluoromethylaniline;
N-(2-n-butoxy-2-p-nitrophenylpropionyl)-4-nitro-3-trifluoromethylaniline;
4-nitro-3-trifluoromethyl-N-(2-p-nitrophenylpropionyl)aniline;
4-nitro-3-trifluoromethyl-N-(2-hydroxy-2-p-methanesulphinylphenylpropionyl)aniline;
4-nitro-3-trifluoromethyl-N-(2-hydroxy-2-p-methanesuiphonylphenylpropionyl)aniline;
4-nitro-3-trifluoromethyl-N-(3-chloro-2-hydroxy-2-p-nitrophenylpropionyl)aniline;
3,4-dichloro-N-(3-chloro-2-hydroxy-2-nitrophenylpropionyl)aniline;
3-chloro-4-cyano-N-(3-chloro-2-hydroxy-2-p-nitrophenylpropionyl)aniline;
3,4-dichloro-N-(2-hydroxy-2-p-methoxycarbonylphenylpropionyl)aniline;
5-methyl-5-p-nitrophenyl-3-(4-nitro-3-trifluoromethylphenyl)oxazolidine-2,4-dione;
3,4-dicyanoisobutyrylanilide;
3,4-dicyano-(2-hydroxy-2-methylpropionyl)anilide;
3,4-dicyano-N-methylisobutyryanilide;
3,4-dicyano-(2-methoxy-2-methylpropionyl)anilide;
4-cyano-3-trifluoromethyl-(2-hydroxy-2-methylpropionyl)anilide;
3,4-dicyano-(2-acetoxy-2-methylpropionyl)anilide;
3,4-dicyano-(2-hydroxy-2-methylpropionyl)anilide;
3,4-dicyano-(cyclopropanecarbonyl)anilide;
3-cyano-4-nitro-(2-hydroxy-2-methylpropionyl)anilide;
3,4-dicyano-N-methylisobutyranilide ;
3,4-dicyano-N-ethylisobutyranilide ;
N-butyl-3,4-dicyanoisobutyranilide ;
3,4-dicyano-(2-decanoyloxy-2-methylpropionyl)anilide ;
3,4-dicyano-(2-hydroxy-2-methylpropionyl )anilide ;
3-chloro-4-cyano-N-(3-ethylthio-2-hydroxy-2-methylpropionyl)aniline;
3-chloro-4-cyano-N- ( 3-ethylsulphonyl-2-hydroxy-2-methylpropionyl )aniline;
4-cyano-3-trifluoromethyl-N-(2-hydroxy-2-methyl-3-phenylsulphonylpropionyl)aniline;
4-cyano-3-trifluoromethyl-N-(3-ethylsulphonyl-2-hydroxy-2-methylproionyl )aniline;
4-nitro-3-trifluoromethyl-N-(2-hydroxy-3-phenylsulphonyl-2-methylpropionyl)aniline;
4-nitro-3-trifluoromethyl-N-(3-ethylsulphonyl-2-hydroxy-2-methylpropionyl)aniline;
3-chloro-4-nitro-N-(2-hydroxy-3-phenylthio-2-methylpropionyl)aniline;
4-nitro-3-trifluoromethyl-N-[2-hydroxy-2-methyl-3-(thiazol-2-ylthio)propionyl]aniline;
4-nitro-3-trifluoromethyl-N-(3-allylthio-2-hydroxy-2-methylpropionyl)aniline;
4-nitro-3-trifluoromethyl-N-(3-p-fluorophenylthio-2-hydroxy-2-methylpropionyl)aniline;
4-nitro-3-trifluoromethyl-N-[2-hydroxy-2-methyl-3-(pyrid-2-ylthio)propionyl]aniline;
4-nitro-3-trifluoromethyl-N-[2-hydroxy-2-methyl-3-(5-methyl-1,3,4-thiadiazol-2-ylthio)propionyl)aniline;
4-nitro-3-trifluoromethyl-N-[2-hydroxy-2-methyl-3-(4-methylthiazol-2-ylthio)propionyl]aniline;
4-nitro-3-trifluoromethyl-N-[2-hydroxy-2-methyl-3-(pyrid-2-ylsulphonyl)propionyl]aniline;
4-nitro-3-trifluoromethyl-N-(3-p-fluorophenyl-sulphonyl-2-hydroxy-2-methylpropionyl)aniline;
4-cyano-3-trifluoromethyl-N-[2-hydroxy-2-methyl-3-(thiazol-2-ylthio)propionyl]aniline;
4-cyano-3-trifluoromethyl-N-[2-hydroxy-2-methyl-3-(pyrid-2-ylthio)propionyl]aniline;
4-cyano-3-trifluoromethyl-N-(2-hydroxy-2-methyl-3-methylthiopropionyl)aniline;
4-cyano-3-trifluoromethyl-N-(3-p-fluorophenylthio-2-hydroxy-2-methylpropionyl)aniline; and
4-cyano-3-trifluoromethyl-N-(3-p-fluorophenyl-sulphonyl-2-hydroxy-2-methylpropionyl)aniline,
and the like.

Typical suitable steroidal antiandrogens known in the art include cyproterone, 6-chloro- 1,2-dihydro- 17-(acetyl)-3'-H-cyclopropa[1,2]-pregna-1,4,6-triene-3,20-dione, available under the tradename of Androcur from Schering A. G., W. Berlin.
See U.S.
Patent 3,234,093.

5

Fertilized poultry embryos, preferably chicken turkey, duck or goose, are treated on about day 0 to about day 9 of incubation with one or more estrogens or antiandrogens or estrogens in combination with one or more antiandrogen compounds. Preincubation as used herein is considered to be day 0, thus day 1 is the day the eggs are placed in the incubator. Incubation begins when the embryos are placed in an incubator. Treatment is by one or more injections of the combination into the embryo or by dipping the embryonated eggs one or more times in a solution of the combination of antiandrogen or antiandrogens and estrogen or estrogens. A single injection is given on about day 0 to about day 9 of incubation. Multiple injections are given every other or every third day starting at about day 0 of incubation and are continued through about day 9 of incubation. Dipping of embryos is by a single treatment or multiple treatments at times discussed above. Dipping as used herein also includes pressure impregnating fertilized eggs with one or more estrogen in combination with one or more antiandrogen. The estrogen, antiandrogen or estrogens and antiandrogens are dissolved or suspended in a physiologically acceptable carrier. Such aqueous physiologically acceptable carriers include, but are not limited to water, saline, physiologic saline, buffer saline, phosphate buffered saline, phosphate buffered saline glucose. Non-aqueous physiologically acceptable carriers include, but are not limited to, 1,2-propane-diol, dimethyl sulfoxide, ethanol and the like.

The concentration of estrogen, antiandrogen or estrogens, antiandrogens is from about 0.1 mg/ml to about 100 mg/ml and the injection volume ranges from about 10 $\mu$l to about 100 $\mu$l producing doses of about 1 $\mu$g to about 10 mg. Injections are made by hand or by machine with about 23 G by 1 in needles. The injection is made under the air sac into the egg white surrounding the developing embryo. Administration of the estrogen in combination with the antiandrogen is also possible by injections made into the air sac without piercing the membrane. Administration is also possible by injection into the opposite end of the egg directly into the albumin. The injection hole is sealed with Scotch tape, wax, glue and the like. The estrogens in combination with the antiandrogens can also be administered by dipping the egg in a solution containing one or more compounds at concentrations as described above. Injected or dipped eggs are placed in a humidified, rocking incubator and allowed to develop and hatch. The active compounds may be administered together or in any order.

The combination of estrogens and antiandrogens will result in the conversion of genotypic males to phenotypic females using lower doses of each compound. The combination will also allow the conversion to occur in a shorter time period than when either compound is used alone. The combination of estrogen and antiandrogen is necessary because it not only alters the endogenous estrogen: androgen ratio which is critical for sex differentiation, but prevents endogenous androgens present in genotypic males from exerting their effects.

The ability to cause a permanent alteration in sex determination is important because this would permit the homogametic male (ZZ) to produce eggs carrying only the Z sex chromosome only. Since normal chicken sperm cells also carry the Z sex chromosome only, fertilization of an egg from a sex reversed male with sperm from a normal male must produce a ZZ or male genotype. All male hatches from crosses of a sex reversed parent with a normal parent have been described for fish. Males are desirable in the poultry broiler industry because they gain weight more quickly, they have a better feed efficiency, and a higher lean:fat ratio than females.

Conversion to female phenotype is determined at about day 14 of incubation, at hatching and posthatching. Pre-hatching conversion is determined by removing the embryos from their shells and dissecting to observe development of the gonads. Female phenotype is determined following hatching by organ sex characterization and/or by vent sexing which techniques are known in the art.

The following examples illustrate the present invention without, however, limiting the same thereto.

## EXAMPLE 1

Phenotypic Conversion Of Rhode Island Red X Plymouth Barred Rock Embryos By Flutamide

Embryonated Rhode Island Red X Plymouth Barred Rock eggs are given single or multiple injections of the antiandrogen 3'-trifluoromethyl-2-methyl-4'-nitropropioanilide (flutamide), dissolved in 0.1 ml of 1,2-propane-diol, on various days of development ranging form day 0 to day 9 of incubation. Single or multiple doses of 0.01 mg to 10 mg are effective in causing sex reversal when sexual phenotype is determined by examination of gonadal morphology on day 15 of incubation. This effect is permanent since genotypic males develop gonadal structures resembling an ovary and oviduct.

EXAMPLE 2

Phenotypic Conversion Of Rhode Island Red X Plymouth Barred Rock Embryos by β-Estradiol and Flutamide

Embryonated Rhode Island Red X Plymouth Barred Rock eggs are given single or multiple injections of the antiandrogen 3'trifluoromethyl-2-methyl-4'-mitropropionanilide (flutamide), and (17)-estra-1,3,5 (10) -triene-3,17-diol (17β-estradiol), dissolved in 0.1 ml 1,2-propane-diol, on various days of development ranging from day 0 to day 9 of incubation. Single or multiple doses of 0.01 mg to 10 mg are effective in causing sex reversal when sexual phenotype is determined by examination of gonadal morphology on day 15 of incubation. The effect is permanent since genotypic males develop gonadal structures resembling an ovary and oviduct.

EXAMPLE 3

Phenotypic Conversion Of Rhode Island Red X Plymouth Barred Rock Embryos By 17 β-estradiol.

Embryonated Rhode Island Red X Plymouth Barred Rock eggs were given a single injection on day 5 of incubation with either 0.1 mg or 1.0 mg of 17 β-estradiol dissolved in 0.1 ml 1,2-propane-diol. Sexual phenotype was determined by examination of gonadal morphology on day 15 of incubation.

## TABLE 1
## FEMINIZATION OF MALE EMBRYOS
## TREATED WITH ESTROGEN

| TREATMENT | MALE | FEMALE | INTERSEX |
|---|---|---|---|
| vehicle | 3 | 3 | 0 |
| 0.1 mg | 0 | 8 | 1 |
| 1.0 mg | 0 | 6 | 2 |

The results demonstrate that male chicken embryos treated with 17 β-estradiol are converted to phenotypic female embryos.

EXAMPLE 4

Phenotypic Conversion Of Arbor Acre X Arbor Acre Embryos By 17 β-estradiol

Phenotypic conversion was further demonstrated by vent sexing hatched embryos after equivalent treatment with 17-beta-estradiol. Embryonated Arbor Acre X Arbor Acre eggs were injected on day 5 of incubation with 0.1 mg of 17-beta-estradiol dissolved in 0.05 ml of 1,2-propane-diol. Chicks were vent sexed and feather sexed one day after hatching.

## TABLE 2

### FEMINIZATION OF HATCHED MALE CHICKS
### TREATED WITH ESTROGEN

| TREATEMNT | VENT SEX | | FEATHER SEX | |
|---|---|---|---|---|
| | MALE | FEMALE | MALE | |
| FEMALE | | | | |
| control | 24 | 16 | 24 | 16 |
| vehicle | 36 | 35 | 35 | 36 |
| 1.0 mg | 0 | 51 | 27 | 24 |

Since feather sex reflects the genotype of the animal, and vent sex indicates the sexual phenotype of the animal, this shows that a single treatment of chicken embryos with estrogens has a permanent effect on sex determination causing a feminization of males. Furthermore, the treatment is effective in both leghorn and broiler lines.

## Claims

1. A method for converting genotypic male poultry into phenotypic female poultry comprising administering to a healthy poultry embryo a phenotypic converting amount of an estrogen which results in the conversion of genotypic male poultry into phenotypic female poultry.

2. A method for converting genotypic male poultry into phenotypic female poultry comprising administering to a healthy poultry embryo a phenotypic converting amount of an estrogen.

3. A method for converting genotypic male poultry into phenotypic female poultry comprising administering to a healthy poultry embryo a phenotypic converting amount of an antiandrogen which results in the conversion of genotypic male poultry into phenotypic female poultry.

4. A method for converting genotypic male poultry into phenotypic female poultry comprising administering to a healthy poultry embryo a phenotypic converting amount of an antiandrogen.

5. A method for converting genotypic male poultry into phenotypic female poultry comprising administering to a healthy poultry embryo a phenotypic converting amount of an estrogen in combination with an antiandrogen which results in the conversion of genotypic male poultry into phenotypic female poultry.

6. A method for converting genotypic male poultry into phenotypic female poultry comprising administering to a healthy poultry embryo a phenotypic converting amount of an estrogen in combination with an antiandrogen.

7. The antiandrogen according to claim 3, claim 4, claim 5 or claim 6 wherein the said antiandrogen is selected from the group consisting of: 1-(3′-trifluoromethyl- 4′nitrophenyl)-4,4-dimethyl-imidazoline-2,5-dione; 4′-nitro-3-trifluoromethylisobutyranilide, N-[4-cyano-3-(trifluoromethyl) phenyl]-3-t(4-fluorophenyl)sulfonyl]-2-hydroxy-2-methylpropionamide and 6-chloro-1,2-dihydro-17-(acetyl)-3′-H-cyclopropa[1,2]pregna-1,4,6-triene-3,20-dione.

8. The estrogen according claim 1, claim 2, claim 5 or claim 6 wherein the said estrogen is selected form the group consisting of: 17 $\beta$-estradiol, (17 $\beta$)-Estra-1,3,5(10)-triene-3,17-diol; estradiol benzoate, (17 $\beta$)-Estra-1,3,5(10)-triene-3,17-diol-3-benzoate; estradiol 17 $\beta$-cypionate, (17 $\beta$)-estra-1,3,5(10)-triene-3,17-diol-17-cyclopentanepropanoate; estrone, 3-hydroxyestra1,3,5(10)-trien-17-one, 1,3,5-estratriene-3,17 $\alpha$-diol and estriol, estra-1,3,5(10)-triene-3,16,17-triol, chlorotris (p-methoxyphenyl) ethylene, 4,4′-(diethylideneethylene) diphenol, trans-4,4′-dihydroxy-$\alpha$ $\beta$-diethylstilbene, 17 $\alpha$-ethynylestra-1,3,5(10)-triene-3,17$\beta$-diol and 3-methoxy-19,nor-17 $\alpha$-pregna-1,3,5(10)-triene-20-yn-17-ol.

9. The method of claim 8 wherein the estrogen is 17 $\beta$-estradiol.

9. The method accroding to claim 1, claim 2, claim 3, claim 4, claim 5 or claim 6 wherein the poultry embryos are treated with the estrogen in combination with antiandrogen on about day 0 to about day 9 of incubation.

10 The method accroding to claim 1, claim 2, claim 5 or claim 6 wherein the poultry embryos are treated with one or more estrogens.

11. The method of claim 3, claim 4, claim 5 or claim 6 wherein poultry embryos are treated one or more times with an antiandrogens.

12. A composition for converting genotypic male poultry into phenotypic female poultry comprising administering to a healthy poultry embryo a phenotypic converting amount of an estrogen in combination with an antiandrogen which results in the conversion of genotypic male poultry into phenotypic female poultry.

13. A composition for converting genotypic male poultry into phenotypic female poultry comprising administering to a healthy poultry embryo a phenotypic converting amount of an antiandrogen which results in the conversion of genotypic male poultry into phenotypic female poultry.

14. A composition for converting genotypic male poultry into phenotypic female poultry comprising administering to a healthy poultry embryo a phenotypic converting amount of an estrogen which results in the conversion of genotypic male poultry into phenotypic female poultry.